# EUROPÄISCHE PATENTANMELDUNG

(11) **EP 0 811 601 A1**
(43) Veröffentlichungstag der Anmeldung: **10.12.1997**
(21) Anmeldenummer: 97107845.6
(22) Anmeldetag: 14.05.1997
(51) Int. Cl.: C07C 69/92, C07C 69/78, C07C 67/11

(54) **Verfahren zur Herstellung von Estern aromatischer Carbonsäuren**

(30) Priorität: 20.05.1996 DE 19620191
(71) Anmelder: Clariant GmbH, 65929 Frankfurt am Main (DE)
(72) Erfinder: Pfirmann, Ralf, Dr., 64347 Griesheim (DE); Papenfuhs, Theodor, Dr., 60433 Frankfurt (DE)

(57) **Zusammenfassung**

Die vorliegende Erfindung betrifft ein Verfahren zur Herstellung von Verbindungen der Formel (1)

R¹R²R³R⁴R⁵ArCOOR (1)

worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OR, NHR, NR₂, SR oder COOR, wobei R ein Alkylrest mit 1 bis 4 Kohlenstoffatomen ist, stehen, Ar für einen Arylrest mit 6 bis 12 Kohlenstoffatomen steht und der in Formel (1) ausgewiesene Rest R die vorstehende Bedeutung hat, indem man eine Verbindung der Formel (2)

R¹R²R³R⁴R⁵ArCOOH (2)

worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OH, NH₂, NHR, SH oder COOH stehen und Ar dieselbe Bedeutung wie in Formel (1) hat, mit einem Sulfat der Formel (RO)₂SO₂, worin R die vorstehende Bedeutung hat, in Anwesenheit eines wasserunlöslichen tertiären Amins und Wasser bei einer Temperatur von 10 bis 120°C in Anwesenheit oder Abwesenheit eines wasserunlöslichen Lösungsmittels und unter Zugabe einer Base bei einem pH-Wert von 5 bis 12 umsetzt.

## Beschreibung

Die vorliegende Erfindung betrifft ein Verfahren zur Herstellung von Estern aromatischer Carbonsäuren durch Alkylierung von aromatischen Carbonsäuren, die gegebenenfalls weitere alkylierbare Substituenten enthalten.

Ester aromatischer Carbonsäuren haben aufgrund ihrer vielseitigen Eigenschaften eine große technische Bedeutung erlangt. Sie lassen sich in unterschiedlichen Bereichen anwenden. Salicylsäureester dienen als Riechstoffe. Phthalsäureester höherer Alkohole werden als Weichmacher für Polyvinylchlorid (PVC) eingesetzt und Phthalsäureester mehrwertiger Alkohole werden zu Herstellung von Lackrohstoffen verwendet. Einige Ester der p-Aminobenzoesäure, beispielsweise der p-Aminobenzoesäureethylester (Anaesthesin) oder der p-Aminobenzoesäure-β-diethylaminoethylester (Procain) haben sich in Form ihrer Hydrochloride als Lokalanaesthetika bewährt (Beyer-Walter, Lehrbuch der organischen Chemie, 21. Auflage, Seiten 553, 559 und 551; S. Hirzel Verlag Stuttgart 1988). Ester von fluorierten Benzoesäuren, beispielsweise Ester der 2,3,4,5-Tetrafluorbenzoesäure (Drugs of the future 1993, Band 18 Heft 8, Seiten 717 bis 720) oder der Methylester der 3-Methoxy-2,4,5-trifluorbenzoesäure (US 5 047 538), lassen sich als Vorprodukte für die Herstellung antibakterieller Mittel aus der Reihe der Fluorchinoloncarbonsäuren verwenden oder in weitere für die Herstellung dieser antibakteriellen Mittel benötigten Vorprodukte umwandeln.

Carbonsäureester lassen sich hauptsächlich auf zwei Wegen herstellen:
1. Durch Veresterung der Carbonsäuren mit einem Überfluß an Alkohol unter sauren Bedingungen, wobei Wasser abgespalten wird.
2. Durch Alkylierung eines Carbonsäuresalzes mittels eines Alkylierungsmittels, beispielsweise eines Alkylhalogenides. Dabei wird das Carbonsäuresalz üblicherweise in Form einer durch Umsetzung der Carbonsäure mit einer wäßrigen Base hergestellten wäßrigen Lösung verwendet oder in situ durch Reaktion der Carbonsäure mit einer in Wasser gelösten Base hergestellt.

J. Barry beschreibt in Synthesis (1985), 40-45 eine Herstellung von aromatischen Carbonsäureestern durch Alkylierung von Kaliumsalzen aromatischer Carbonsäuren ohne Zusatz eines Lösungsmittels, jedoch unter Verwendung eines Phasen-Transfer-Katalysators. Neben Alkylhalogeniden werden Dimethylsulfat und Diethylsulfat als Alkylierungsmittel eingesetzt.

Das Kaliumcarboxylat wird entweder durch Auflösen der Carbonsäure in der stöchiometrischen Menge einer wäßrigen Kaliumhydroxidlösung, nachfolgende Verdampfung des Wassers und Vermahlen des trocknen Kaliumcarboxylats zu einem feinen Pulver (Methode A) oder durch Vermischen von feinverteilter Carbonsäure, feinverteiltem Kaliumhydroxid und des als Phasen-Transfer-Katalysators eingesetzten Ammoniumsalzes, durch nachfolgendes Erhitzen dieses Gemisches auf 140°C und abschließendes Vermahlen des Schmelzkuchens (Methode B) hergestellt.

Das nach Methode A hergestellte Kaliumcarboxylat wird mit dem Phasen-Transfer-Katalysator und anschließend mit dem Alkylierungsmittel beispielsweise Dimethylsulfat, versetzt, während das nach Methode B hergestellte Kaliumcarboxylat, das den Phasen-Transfer-Katalysator bereits enthält, direkt mit dem Alkylierungsmittel versetzt wird.

Die Mischung wird geschüttelt, unter den angegebenen Reaktionsbedingungen zur Umsetzung gebracht, anschließend zweimal mit Ether verdünnt, über eine kurze mit einem Hilfsmittel gefüllte Kolonne filtriert und anschließend durch Chromatographie oder Kristallisation gereinigt.

Nach dieser Methode lassen sich auch aromatische Carbonsäuren, die gegebenenfalls weitere alkylierbare Substituenten enthalten, unter Verwendung beispielsweise von Dimethylsulfat umsetzen.

Das vorstehend beschriebene Verfahren weist mehrere Nachteile auf. Zum einen erfordert sowohl das nach Methode A als auch nach Methode B hergestellte Kaliumcarboxylat einen beträchtlichen Arbeitsaufwand, beispielsweise das Verdampfen von Wasser und das mechanische Vermahlen der Carbonsäure, des Kaliumhydroxids und des Kaliumcarboxylat-Kuchens. Zum anderen erweist sich die Aufarbeitung des anfallenden Reaktionsgemisches (zweimaliges Verdünnen mit Ether, Filtration und nachfolgende Reinigung durch Säulenchromatographie oder Kristallisation) als recht umständlich.

Darüberhinaus gelangen die als Phasen-Transfer-Katalysator verwendeten Ammoniumsalze in das Abwasser, belasten dieses und führen, da sie schwer abzubauen sind, zu Problemen in der Abwasseraufarbeitung.

Es stellt somit eine lohnende Aufgabe dar, ein Verfahren zur Herstellung von aromatischen Carbonsäureestern bereitzustellen, das die vorstehend geschilderten Nachteile nicht aufweist und das es zudem gestattet, neben der Alkylierung der Carboxylgruppe auch weitere alkylierbare Substituenten zu alkylieren.

Diese Aufgabe wird gelöst durch ein Verfahren zur Herstellung von Verbindungen der Formel (1)

R¹R²R³R⁴R⁵ArCOOR (1)

worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OR, NHR, NR₂, SR oder COOR, wobei R ein Alkylrest mit 1 bis 4 Kohlenstoffatomen ist, stehen, Ar für einen Arylrest mit 6 bis 12 Kohlenstoffatomen steht und der in Formel (1) ausgewiesene Rest R die vorstehende Bedeutung hat. Es ist dadurch gekennzeichnet, daß man eine Verbindung der Formel (2)

R¹R²R³R⁴R⁵ArCOOH (2)

worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OH, NH₂, NHR, SH oder COOH stehen und Ar dieselbe Bedeutung wie in Formel (1) hat, mit einem Sulfat der Formel (RO)₂SO₂, worin R die vorstehende Bedeutung hat, in Anwesenheit eines wasserunlöslichen tertiären Amins und Wasser bei einer Temperatur von 10 bis 120°C in Anwesenheit oder Abwesenheit eines wasserunlöslichen Lösungsmittels und unter Zugabe einer Base bei einem pH-Wert von 5 bis 12 umsetzt.

Das erfindungsgemäße Verfahren weist mehrere Vorteile auf.

Zum einen ist es nicht erforderlich, die umzusetzende aromatische Carbonsäure in Form ihrer wasserfreien Kaliumsalze einzusetzen, sondern es genügt, aus der aromatischen Carbonsäure in situ eine wäßrige Lösung eines Salzes der aromatischen Carbonsäure herzustellen.
Zum anderen ist es in einer Vielzahl von Fällen nicht notwendig, einen schwer abbaubaren Phasen-Transfer-Katalysator einzusetzen. Dadurch wird auch eine Belastung des Abwassers und eine Störung der Aufarbeitung des Abwassers vermieden.

Ein weiterer Vorteil besteht darin, daß man nach Beendigung der Umsetzung lediglich die organische Phase, die das wasserunlösliche tertiäre Amin und das Wertprodukt enthält, von der wäßrigen Phase trennen muß. Die weitere Aufarbeitung erfolgt in der Regel durch Destillation. Dadurch wird die Anwendung eines problematischen Lösungsmittels, beispielsweise Ether, vermieden.

Darüberhinaus ist es als überraschend anzusehen, daß das wasserunlösliche tertiäre Amin mit dem Dialkylsulfat überhaupt nicht oder nur in recht geringem Umfange reagiert. Dies hat zur Folge, daß das erfindungsgemäße Verfahren nicht zu einem erhöhten Verbrauch von Dialkylsulfat führt.

Von Vorteil ist auch, daß man nach Abtrennung des Wertproduktes also der Verbindungen der Formel (1), das verbleibende wasserunlösliche tertiäre Amin wieder in die Reaktion einsetzen kann. Dadurch wird auch der Bedarf an Hilfsstoffen, die zu einer zusätzlichen Belastung des Abwassers führen können, niedrig gehalten.

Das erfindungsgemäße Verfahren ermöglicht es nicht nur, die Carboxylgruppe in der Verbindung der Formel (2), sondern auch weitere in der aromatischen Carbonsäure der Formel (2) vorhandene, alkylierbare Gruppen nämlich die OH-, NH₂-, NHR-, SH- und COOH-Gruppe, zu alkylieren. Falls gewünscht, läßt sich die Alkylierung dieser unterschiedlich reaktiven Gruppen auch bei einem einzigen pH-Wert durchführen. Dadurch gestaltet sich das erfindungsgemäße Verfahren recht einfach.

Man kann aber auch die Umsetzung bei unterschiedlichen pH-Werten ablaufen lassen, um zunächst bei einem höheren pH-Wert und daran anschließend bei einem niedrigeren pH-Wert zu arbeiten. Auch diese Variante des Verfahrens ist recht einfach durchzuführen, da die Umsetzung hierfür nicht unterbrochen werden muß, sondern im gleichen Reaktionsmilieu durchgeführt werden kann.

Üblicherweise setzt man eine Verbindung (2), worin R¹ und R² gleich oder verschieden sind und für Wasserstoff, eine Alkylgruppe mit 1 bis 6 Kohlenstoffatomen, für OH, NH₂ oder COOH, insbesondere für Wasserstoff, für OH oder COOH stehen, ein.

Man kann auch eine Verbindung der Formel (2), worin R¹ oder R² für OH oder COOH, insbesondere für OH steht, einsetzen. Die übrigen vier Reste R¹, R³, R⁴, R⁵ oder R², R³, R⁴, R⁵ sind in diesem Falle gleich oder verschieden und stehen für Wasserstoff, ein Halogen, eine Alkyl- oder Alkoxygruppe mit 1 bis 6 Kohlenstoffatomen, insbesondere für Wasserstoff, Fluor, Chlor oder eine Alkylgruppe mit 1 bis 6 Kohlenstoffatomen, bevorzugt für Wasserstoff oder Fluor.

In einer Reihe von Fällen kann man eine Verbindung der Formel (2), worin R³, R⁴ und R⁵ gleich oder verschieden sind und für Wasserstoff, ein Halogen, eine Alkyl- oder eine Alkoxygruppe mit 1 bis 6 Kohlenstoffatomen, insbesondere für Wasserstoff, Fluor, Chlor oder eine Alkylgruppe mit 1 bis 6 Kohlenstoffatomen, bevorzugt für Wasserstoff oder Fluor stehen, in die Reaktion einsetzen.

Man setzt in die Umsetzung eine Verbindung der Formel (2), worin Ar, wie eingangs bereits erwähnt, für einen Arylrest mit 6 bis 12 Kohlenstoffatomen, insbesondere für einen Phenylrest, Biphenylrest oder Naphthylrest, bevorzugt für einen Phenylrest steht, ein.

Ohne Anspruch auf Vollständigkeit seien als Beispiele für Verbindungen der Formel (2)
2-Chlorbenzoesäure,
3-Chlorbenzoesäure,
4-Chlorbenzoesäure,
2-Fluorbenzoesäure,
3-Fluorbenzoesäure,
4-Fluorbenzoesäure,
2-Brombenzoesäure,
3-Brombenzoesäure,
4-Brombenzoesäure,
2,4-Dichlorbenzoesäure
2,4-Difluorbenzoesäure,
3,4-Difluorbenzoesäure,
3,4-Dichlorbenzoesäure,
2,5-Dichlorbenzoesäure,
2,6-Difluorbenzoesäure,
2,3,6-Trifluorbenzoesäure,
2,4,5-Trifluorbenzoesäure,
2,4,5-Trichlorbenzoesäure,
2,3,4,5-Tetrachlorbenzoesäure,
2,3,4,5-Tetrafluorbenzoesäure,
2,3,5,6-Tetrafluorbenzoesäure,
Pentafluorbenzoesäure,
Pentachlorbenzoesäure,
2-Chlor-3,4,5-trifluorbenzoesäure,
2,3-Dichlor-4,5-difluorbenzoesäure,
2,4,5-Trifluor-3-chlorbenzoesäure,
2,4-Difluor-3,5-dichlorbenzoesäure,
2,6-Difluor-3,5-dichlorbenzoesäure,
2-Hydroxybenzoesäure,
3-Hydroxybenzoesäure,
4-Hydroxybenzoesäure,
2-Chlor-4-hydroxybenzoesäure,
2-Fluor-4-hydroxybenzoesäure,
2,3,5-Trifluor-4-hydroxybenzoesäure,
2,4,5-Trifluor-3-hydroxybenzoesäure,
4-Hydroxy-2,3,5,6-tetrafluorbenzoesäure,
5-Chlor-2-hydroxybenzoesäure,
5-Fluor-2-hydroxybenzoesäure,
4-Chlor-2-hydroxybenzoesäure,
5-Chlor-2-hydroxybenzoesäure,
4-Chlor-2-aminobenzoesäure,
4-Fluor-2-aminobenzoesäure,
5-Fluor-2-aminobenzoesäure,
5-Chlor-2-aminobenzoesäure,
3-Amino-2,4,5-trifluorbenzoesäure,
4-Aminobenzoesäure,
4-Amino-2-chlorbenzoesäure,
4-Amino-2-fluorbenzoesäure,
4-Amino-2,3,5-trifluorbenzoesäure,
6-Methyl-3-amino-2,4,5-trifluorbenzoesäure,
3-Hydroxy-2,4-difluorbenzoesäure,
4-Hydroxy-3-fluorbenzoesäure,
4-Hydroxy-3-chlorbenzoesäure,
4-Hydroxy-3,5-dichlorbenzoesäure,
4-Hydroxy-3,5-difluorbenzoesäure,
3-Hydroxytetrafluorbenzoesäure,
2-Hydroxytetrafluorbenzoesäure,
3-Methyl-2,4,5-trifluorbenzoesäure,
3-Ethyl-2,4,5-trifluorbenzoesäure und
6-Methyl-3-hydroxy-2,4,5-trifluorbenzoesäure,
insbesondere 2,3,6-Trifluorbenzoesäure, 2,4,5-Trifluorbenzoesäure, 2,4,5-Trichlorbenzoesäure, 2,3,4,5-Tetrachlorbenzoesäure, 2,3,4,5-Tetrafluorbenzoesäure, 2,3,5,6-Tetrafluorbenzoesäure, 2-Hydroxybenzoesäure, 3-Hydroxybenzoesäure, 4-Hydroxybenzoesäure, 2-Chlor-4-hydroxybenzoesäure, 2-Fluor-4-hydroxybenzoesäure, 2,3,5-Trifluor-4-hydroxybenzoesäure, 2,4,5-Trifluor-3-hydroxybenzoesäure, 4-Hydroxy-2,3,5,6-tetrafluorbenzoesäure, 5-Chlor-2-hydroxybenzoesäure, 5-Fluor-2-hydroxybenzoesäure, 4-Chlor-2-hydroxybenzoesäure, 5-Chlor-2-hydroxybenzoesäure, 3-Hydroxy-2,4-difluorbenzoesäure, 4-Hydroxy-3-fluorbenzoesäure, 4-Hydroxy-3-chlorbenzoesäure, 4-Hydroxy-3,5-dichlorbenzoesäure, 4-Hydroxy-3,5-difluorbenzoesäure, 3-Hydroxytetrafluorbenzoesäure, 2-Hydroxytetrafluorbenzoesäure, 6-Methyl-3-hydroxy-2,4,5-trifluorbenzoesäure genannt.

Man setzt die Verbindung der Formel (2) mit einem Sulfat der Formel (RO)₂SO₂, worin R für einen Alkylrest mit 1 bis 4 Kohlenstoffatomen steht, um. Man setzt als Sulfat der Formel (RO)₂SO₂ insbesondere Dimethylsulfat, Diethylsulfat oder Dibutylsulfat, bevorzugt Dimethylsulfat oder Diethylsulfat ein.

Man läßt die Reaktion in Anwesenheit eines wasserunlöslichen tertiären Amins ablaufen. Unter dem Begriff wasserunlösliches tertiäres Amin werden solche Amine verstanden, die sich in Wasser entweder nur in geringem Umfange oder gar nicht lösen.

Üblicherweise setzt man als wasserunlösliches tertiäres Amin ein Trialkylamin mit 4 bis 20 Kohlenstoffatomen je Alkylrest, ein Gemisch dieser Trialkylamine, eine N-enthaltende heterocyclische Verbindung oder ein Gemisch der vorstehenden Amine, insbesondere ein Trialkylamin mit 6 bis 14 Kohlenstoffatomen je Alkylrest, ein Gemisch dieser Trialkylamine, ein gegebenenfalls alkyliertes Pyridin oder Chinolin, beispielsweise Collidin, Lutidin oder ein Picolin, oder ein Gemisch dieser tertiären Amine ein. Die vorstehend genannten Trialkylamine enthalten geradkettige und/oder verzweigte Alkylreste. Die Alkylreste können gleich oder verschieden sein. Gut geeignet sind Gemische der vorstehend genannten Trialkylamine.

In einer Reihe von Fällen hat sich ein Gemisch von Trialkylaminen mit 6 bis 12 Kohlenstoffatomen je Alkylrest, die gleiche oder verschiedene geradkettige oder verzweigte Alkylreste enthalten, als besonders geeignet erwiesen.

Da die Umsetzung in Anwesenheit des wasserunlöslichen tertiären Amins und Wasser durchgeführt wird, läuft die Umsetzung in einem aus zwei flüssigen Phasen bestehenden Reaktionsmedium ab. Um die Umsetzung zu begünstigen, ist für eine gute Durchmischung der beiden Phasen zu sorgen. Nach Beendigung der Umsetzung entmischen sich üblicherweise die beiden Phasen, so daß die Trennung der wäßrigen Phase von der organischen Phase keine Probleme bereitet.

Ohne Anspruch auf Vollständigkeit zu erheben, seien als wasserunlösliche Trialkylamine der vorstehend genannten Art folgende Verbindungen genannt: Tri-n-butylamin, Triisobutylamin, Tri-n-pentylamin, Triisopentylamin, Tri-n-hexylamin, Tri-isohexylamin, Tri-n-heptylamin, Triisoheptylamin, Tri-n-octylamin, Triisoctylamin, Tri-n-decylamin, Triisodecylamin, Tri-n-dodecylamin, Triisododecylamin, Trialkylamine mit geraden und/oder verzweigten Ketten mit 6 bis 14 Kohlenstoffatomen, Pyridin, α-Picolin, β-Picolin, γ-Picolin, 2,4-Dimethylpyridin(α,γ-Lutidin), 2,6-Di-tert.-butylpyridin, 2,4,6-Trimethylpyridin, (Collidin), Triethylpyridin, Chinolin, Methylchinoline, Ethylchinoline, gemischte Amine wie Butyldihexylamin, Dioctyldecylamin, Hexyldioctylamin, Dihexyloctylamin, Diheptyloctylamin, Didecyloctylamin, Didodecyloctylamin, Didodecyldecylamin, Didecyldodecylamin, Dioctyldodecylamin, Dinonyloctylamin, Dinonyldecylamin, Dinonyldodecylamin sowie deren Gemische.

In einer Vielzahl von Fällen hat es sich als ausreichend erwiesen, die Umsetzung bei 20 bis 80°C, insbesondere bei 30 bis 60°C durchzuführen.

Man führt die Umsetzung in Anwesenheit oder Abwesenheit eines wasserunlöslichen Lösungsmittels durch. Man kann als wasserunlösliches Lösungsmittel einen aliphatischen Kohlenwasserstoff, einen halogenierten aliphatischen Kohlenwasserstoff, einen aromatischen Kohlenwasserstoff, einen halogenierten aromatischen Kohlenwasserstoff, einen aromatischen Ether oder ein Gemisch dieser Lösungsmittel einsetzen. Ohne Anspruch auf Vollständigkeit zu erheben, seien an dieser Stelle als wasserunlösliche Lösungsmittel Hexan, Heptan, Oktan, Dichlormethan, Trichlormethan, Toluol, o-Xylol, m-Xylol, p-Xylol, Gemische isomerer Xylole, Ethylbenzol, Butylbenzol, Chlorbenzol, o-Dichlorbenzol, m-Dichlorbenzol, p-Dichlorbenzol, Chlortoluol, m-Chlortoluol, p-Chlortoluol, Diphenyl, Diphenylmethan oder Diphenylether genannt. Es lassen sich auch Mischungen dieser Lösungsmittel verwenden.

Die Umsetzung läßt sich in Abwesenheit oder Anwesenheit eines Phasentransferkatalysators durchführen.

In einzelnen Fällen kann es, beispielsweise zum Zweck der Verbesserung der Durchmischung, Minimierung des Einsatzes und Verbrauch an Dialkylsulfat und/oder Steigerung der Reaktionsgeschwindigkeit, hilfreich sein, die Umsetzung in Anwesenheit eines Phasentransferkatalysators durchzuführen. Üblicherweise verwendet man als Phasentransferkatalysator ein quartäres Ammonium- oder Phosphoniumsalz oder Gemische derselben, insbesondere ein quartäres Ammoniumsalz der allgemeinen Formel 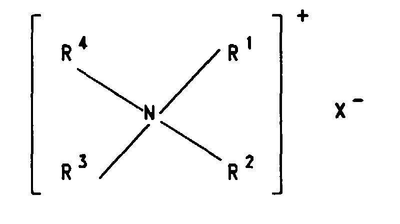 worin R¹, R², R³ und R⁴ gleich oder verschieden sind und für Kohlenwasserstoffreste mit insgesamt 10 bis 50 Kohlenstoffatomen stehen und X⁻ein Halogenidion, Hydrogensulfation oder Hydroxylion, insbesondere ein Chlorid-, Bromid- oder Hydrogensulfation bedeutet, oder ein Gemisch derartiger quartärer Ammoniumsalze.

Geeignet als Phasentransferkatalysator sind Tetra(C₁-C₂₀)alkylammoniumsalze, Tri(C₁-C₂₀)alkylbenzylammoniumsalze, Di(C₁-C₂₀)alkyldibenzylammoniumsalze, deren Benzylrest unsubstitutiert oder durch Cl, Br, (C₁-C₄)Alkyl, (C₁-C₄)Alkoxy substituiert, insbesondere unsubstituiert, ist.

Als Phasentransferkatalysatoren lassen sich solche, die beispielsweise in DE-OS 2 634 419, DE-OS 3 120 912 und DE-OS 3 737 919 beschrieben sind, beispielsweise Tetrabutylammoniumbromid, Tetramethylammoniumchlorid, Tetramethylammoniumhydrogensulfat, Benzyldodecyldimethylammoniumchlorid, Stearyldimethylbenzylammoniumhalogenid, Hexadecyltrimethylammoniumhalogenid, ein einen oder mehrere, insbesondere einen oder zwei, Cocos(C₁₀-C₁₈)alkyl-Reste enthaltendes quartäres Ammoniumhalogenid, beispielsweise Dicocos(C₁₀-C₁₈)alkyldimethylammoniumhalogenid, Dimethylbenzylcocos(C₁₀-C₁₈)alkylammoniumhalogenid, wobei das Halogenid insbesondere Chlorid oder Bromid ist, einsetzen. Besonders bewährt hat sich dabei Dimethylbenzylcocos(C₁₀-C₁₈)alkylammoniumchlorid mit einem durchschnittlichen Molekulargewicht von 382,5, das bevorzugt als 50 %ige wäßrige Lösung verwendet wird (Dodigen 226).

Man setzt den Phasentransferkatalysator üblicherweise in einer Menge von 0,05 bis 10, insbesondere 0,2 bis 2,5, bevorzugt 0,25 bis 1,5 Gew.-%, bezogen auf die wäßrige Phase, ein.

Man setzt als Base eine wäßrige Lösung und/oder eine Suspension eines Alkalihydroxids oder Erdalkalihydroxids, insbesondere eine wäßrige LiOH, NaOH oder KOH Lösung, bevorzugt eine wäßrige NaOH oder KOH Lösung, oder ein Gemisch dieser wäßrigen Lösungen ein.
Die wäßrige Lösung und/oder Suspension enthält üblicherweise 5 bis 50, insbesondere 10 bis 40, bevorzugt 20 bis 35 Gew.-% Alkalihydroxid oder Erdalkalihydroxid.

Für eine Reihe von Fällen hat es sich als nützlich erwiesen, die Umsetzung bei einem pH-Wert von 6 bis 10, insbesondere bei einem pH-Wert von 7 bis 8,5 durchzuführen.

Unter Einhalten der vorstehend genannten Reaktionsbedingungen läßt sich das Verfahren ohne großen technischen Aufwand durchführen.

Man legt in beliebiger Reihenfolge Wasser, die aromatische Carbonsäure (Verbindung der Formel (2)), das wasserunlösliche tertiäre Amin und gegebenenfalls das wasserunlösliche Lösungsmittel vor und stellt anschließend unter Rühren durch Zugabe von Base den gewünschten pH-Wert ein. Man kann auch eine wäßrige Lösung der aromatischen Carbonsäure oder eine wäßrige Lösung eines Salzes der aromatischen Carbonsäure einsetzen. Es ist auch möglich, ein aus Wasser, der aromatischen Carbonsäure, dem wasserunlöslichen tertiären Amin und gegebenenfalls dem wasserunlöslichen Lösungsmittel bestehendes Gemisch, das beispielsweise aus einem vorangegangenen Reaktionsschritt entstammt, in die Reaktion einzusetzen. In diesem Falle stellt man den gewünschten pH-Wert ebenfalls unter Rühren durch Zugabe von Base ein.

Anschließend setzt man das Sulfat der Formel (RO)₂SO₂ und die Base in dem Maße zu, daß der vorgegebene pH-Wert eingehalten wird.

Das wasserunlösliche tertiäre Amin läßt eine Anwendung in einem weiten Mengenbereich zu. Üblicherweise setzt man das wasserunlösliche tertiäre Amin und die aromatische Carbonsäure (Verbindung der Formel (2)) in einem Molverhältnis von (0,01 bis 10):1,insbesondere (0,05 bis 3):1, bevorzugt (0,05 bis 1):1, besonders bevorzugt (0,1 bis 0,5):1, ein.
Der Anteil Wasser ist in weiten Grenzen wählbar.
Das Verhältnis des Volumens der wäßrigen Phase zum Volumen der organischen Phase beträgt üblicherweise (0,05 bis 50):1, insbesondere (0,05 bis 20):1, bevorzugt (0,1 bis 10):1.

In diesem Zusammenhang sei darauf hingewiesen, daß die organische Phase nicht nur das wasserunlösliche tertiäre Amin sondern auch das Wertprodukt, nämlich die Verbindung der Formel (1) und gegebenenfalls das wasserunlösliche Lösungsmittel enthält.

Man setzt das wasserunlösliche Lösungsmittel und die aromatische Carbonsäure (Verbindung der Formel (2)) im Gewichtsverhältnis von (0,05 bis 100):1, insbesondere (0,3 bis 10):1, bevorzugt (0,8 bis 5):1, ein.

Das Sulfat der Formel (RO)₂SO₂ wird, bezogen auf jede in die Verbindung der Formel (2) einzuführende Gruppe R, im Verhältnis (1 bis 10):1, insbesondere (1,1 bis 5):1, bevorzugt (1,2 bis 1,5):1 eingesetzt.
Enthält das Einsatzmaterial außer der Verbindung der Formel (2) noch weitere Stoffe, die mit dem Sulfat reagieren, so ist die Menge an Sulfat entsprechend zu erhöhen.

Es versteht sich von selbst, daß bei der Handhabung des Sulfats, insbesondere bei Handhabung von Dialkylsulfaten, respektive Dimethylsulfat, die entsprechenden Sicherheitsmaßnahmen zu beachten sind.
Je Mol Sulfat der Formel (RO)₂SO₂, das mit der Verbindung der Formel (2) umgesetzt wird, werden 1 bis 1,5, insbesondere 1,01 bis 1,2, bevorzugt 1,01 bis 1,1 Äquivalente Base eingesetzt.
Nach Beendigung der Umsetzung sorgt man dafür, daß noch vorhandenes Sulfat, beispielsweise durch Zugabe wäßriger Lauge, wäßrigen Ammoniaks oder einer wäßrigen Ammoniumsalzlösung zersetzt wird.

Anschließend trennt man die organische Phase, die das Wertprodukt enthält, von der wäßrigen Phase und arbeitet die organische Phase beispielsweise durch Destillation auf.

Falls gewünscht, kann man aber auch das wasserunlösliche Lösungsmittel nach Beendigung der Umsetzung hinzufügen, um beispielsweise die Phasentrennung zu erleichtern.
Das Verfahren läßt sich kontinuierlich oder diskontinuierlich durchführen. Es gestattet, sowohl unter reduziertem Druck als auch bei Atmosphärendruck oder Überdruck zu arbeiten.

Die nachfolgenden Beispiele beschreiben die vorliegende Erfindung, ohne sie darauf zu beschränken.

### Experimenteller Teil

### Beispiel 1

### Herstellung von 4-Hydroxybenzoesäuremethylester und 4-Methoxybenzoesäuremethylester

Die Umsetzung verläuft nach folgendem Reaktionsschema:

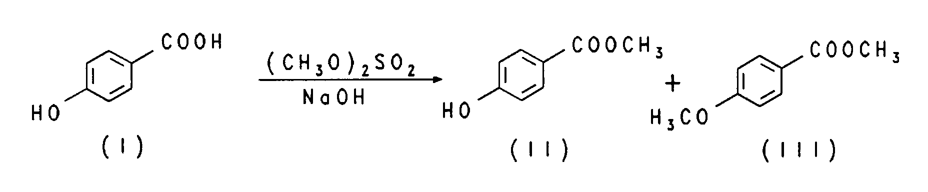

In einem Glaskolben werden 34,5 g (0,25 mol) 4-Hydroxybenzoesäure (I) und 150 g Wasser vorgelegt und vermischt. Die 4-Hydroxybenzoesäure wird zum Teil gelöst, wobei sich eine milchig trübe Suspension bildet. Man löst 5 g eines Gemisches von Trialkylaminen mit 8 bis 10 Kohlenstoffatomen je Alkylrest (Hostarex A327, ein Handelsprodukt der HOECHST AG) in 30 g Xylol und setzt diese Lösung der Suspension zu und erwärmt auf 45°C. Mittels einer geeichten pH-Elektrode, die in die wäßrige Phase eintaucht, wird der pH-Wert kontrolliert. Man stellt durch tropfenweise Zugabe einer 10 gew.-%igen wäßrigen NaOH-Lösung den pH-Wert von 8 ein. Anschließend setzt man innerhalb von 3 Stunden tropfenweise 88,2 g (0,7 mol) Dimethylsulfat unter intensivem Rühren zu. Der pH-Wert wird durch tropfenweise Zugabe von wäßriger NaOH (10 Gew.-%) in einem Bereich von 7,5 bis 8,5 gehalten. Nach Abschluß der Zugabe von Dimethylsulfat wird über Nacht gerührt. Es bilden sich zwei Phasen; eine obere organische Phase, die das Wertprodukt (Gemisch aus 4-Hydroxybenzoesäuremethylester(II) und 4-Methoxybenzoesäuremethylester(III)) enthält, und eine untere, wäßrige Phase.

Die organische Phase wird abgetrennt. Sie enthält nach HPLC-Analyse neben 15 % Lösungsmitteln (Xylol und tertiäre Amine) 40 % (entsprechend 14,7 g ≙ 0,089 mol; 35,4 % der Theorie) 4-Methoxybenzoesäuremethylester und 44 % (entsprechend 16,2 g ≙ 0,106 mol; 42,4% der Theorie), 4-Hydroxybenzoesäuremethylester.
In der Wasserphase befinden sich - bestimmt als HPLC-Flächen-%, gerechnet ohne Wasser und ohne Salzanteile - 51 % Ausgangsmaterial (4-Hydroxybenzoesäure), 37 % 4-Hydroxybenzoesäuremethylester und 11,6 % 4-Methoxybenzoesäuremethylester. Nebenprodukte werden nur in recht geringem Umfange (< 1 %) gefunden.
Die Selektivität der Bildung von (4-Hydroxybenzoesäuremethylester + 4-Methoxybenzoesäuremethylester) beträgt ≥ 95 %.

Führt man die Umsetzung bei 35 bis 40°C unter Konstanthalten des pH-Wertes auf 7 bis 7,5 unter Verwendung der vorstehend genannten Einsatzstoffe und Mengen durch, so erhält man 77.2 g organische Phase. Diese organische Phase enthält (bestimmt mittels HPLC-Analyse) 39,4 g (0,239 mol ≙ 95,4 % der Theorie) 4-Methoxybenzoesäuremethylester und 0,8 g (0,005 mol ≙ 2,1 % der Theorie) 4-Hydroxybenzoesäuremethylester.
Die wäßrige Phase enthält nur noch Spuren von 4-Methoxybenzoesäure.

### Beispiel 2

### Herstellung von 3-Methoxy-2,4,5-trifluorbenzoesäuremethylester

a) Herstellung von 3-Hydroxy-2,4,5-trifluorbenzoesäure durch Decarboxylierung von 4-Hydroxy-3,5,6-trifluorphthalsäure
   Die Umsetzung verläuft nach folgender Gleichung: 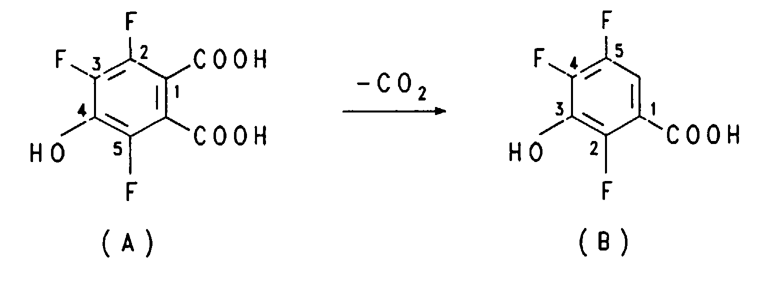
   In einem Glaskolben werden unter Rühren 255 g einer wäßrigen Lösung, die 20,6 g (87,3 mmol) 4-Hydroxy-3,5,6-trifluorphthalsäure (A) enthält vorgelegt und mit 20 g eines Gemisches von Trialkylaminen mit 8 bis 10 Kohlenstoffatomen je Alkylrest (Hostarex A327; ein Handelsprodukt der HOECHST AG) versetzt. Man setzt unter guter Vermischung 49 g einer 30 gew.-%igen wäßrigen Salzsäure zu. Mittels einer geeichten pH-Elektrode, die in die wäßrige Phase eintaucht, wird der pH-Wert kontrolliert. Nach Zugabe der Salzsäure beträgt der pH-Wert 5. Anschließend erhitzt man unter guter Vermischung auf 105°C, hält den pH-Wert durch Zugabe von insgesamt 13,5 g einer 30 gew.-%igen wäßrigen Salzsäure konstant bei pH = 6 und läßt 7 Stunden reagieren. Infolge der dabei ablaufenden Decarboxylierung wird die 4-Hydroxy-3,5,6-trifluorphthalsäure (A) zur 3-Hydroxy-2,4,5-trifluorbenzoesäure (B) umgesetzt.
b) Herstellung von 3-Methoxy-2,4,5-trifluorbenzoesäuremethylester
   Die Umsetzung verläuft nach folgender Gleichung 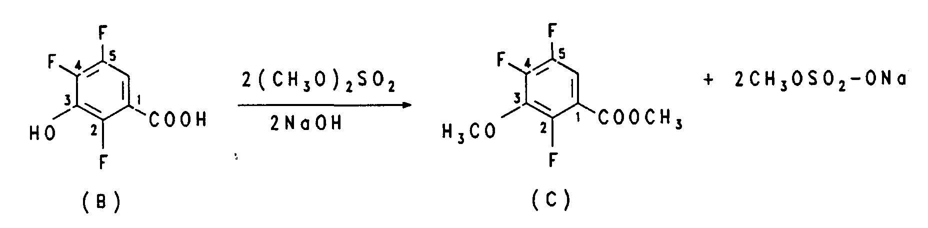
   Man versetzt unter Rühren das aus Beispiel 2a) erhaltene Reaktionsgemisch, das die 3-Hydroxy-2,4,5-trifluorbenzoesäure (B) enthält, mit 34 g einer 10 gew.-%igen wäßrigen Natronlauge. Mittels einer geeichten pH-Elektrode, die in die wäßrige Phase eintaucht, wird der pH-Wert des das Wasser, das Gemisch wasserunlöslicher Trialkylamine mit 8 bis 10 Kohlenstoffatomen je Alkylrest (Hostarex A 327) und die aromatische Carbonsäure, nämlich die 3-Hydroxy-2,4,5-trifluorbenzoesäure, enthaltenden Reaktionsgemisches kontrolliert. Nach Zugabe der Natronlauge beträgt der pH-Wert 7. Anschließend tropft man bei einer Temperatur von 40°C über einen Zeitraum von 80 Minuten insgesamt 90 g (0,72 mol) Dimethylsulfat zu und hält durch Zugabe von insgesamt 37 g einer 10 gew.-%igen wäßrigen Natronlauge den pH-Wert konstant auf 7. Anschließend setzt man 20 g Diphenylmethan zu und trennt die organische Phase (50 g) von der wäßrigen Phase ab.
   Die organische Phase enthält, bestimmt durch kalibrierte (HPLC) flüssigchromatographische Analyse, 17,6 g (80 mmol) 3-Methoxy-2,4,5-trifluorbenzoesäuremethylester entsprechend 91,6 % Ausbeute über 2 Stufen, bezogen auf eingesetzte 4-Hydroxy-3,5,6-trifluorphthalsäure.
c) Wiederholt man Beispiel 2b), setzt jedoch bereits während der Methylierung die vorstehend genannte Menge Diphenylmethan zu, so erhält man 17,4 g (79 mmol) 3-Methoxy-2,4,5-trifluorbenzoesäuremethylester entsprechend 90,5 % Ausbeute, bezogen auf eingesetzte 4-Hydroxy-3,5,6-trifluorphthalsäure.

### Vergleichsbeispiel

### Herstellung von 3-Methoxy-2,4,5-trifluorbenzoesäuremethylester ohne Zusatz eines wasserunlöslichen tertiären Amins

Die Umsetzung verläuft nach folgendem Reaktionsschema:

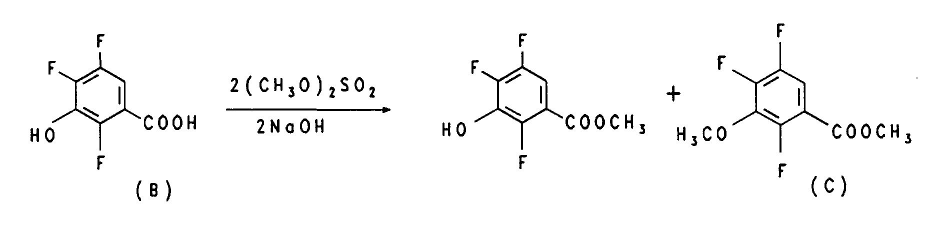

In einem Glaskolben werden unter Rühren 299 g einer wäßrigen Lösung, die 15,6 g (81 mmol) 3-Hydroxy-2,4,5-trifluorbenzoesäure (B) enthält, vorgelegt und werden anstelle des wasserunlöslichen Trialkylamins bzw. Amingemisches mit 10 g Xylol versetzt. Man setzt eine 30 gew.-%ige wäßrige Salzsäure bis zu einem pH-Wert von 7 zu und kontrolliert den pH-Wert mittels einer geeichten pH-Elektrode, die in die wäßrige Phase eintaucht. Anschließend tropft man bei einer Temperatur von 40°C über einen Zeitraum von 3,5 Stunden insgesamt 156,8 g (1,41 mol) Dimethylsulfat zu und hält durch Zugabe von insgesamt 46,6 g einer 10 gew.-%igen wäßrigen Natronlauge den pH-Wert konstant auf 7. Trotz eines beträchtlichen Dimethylsulfat-Überschusses hat sich das dimethylierte Produkt, nämlich der 3-Methoxy-2,4,5-trifluorbenzoesäuremethylester, nur in geringen Mengen gebildet. Man findet die 3-Hydroxy-2,4,5-trifluorbenzoesäure (B) entsprechend einer Ausbeute von 8,6 %, den 3-Hydroxy-2,4,5-trifluorbenzoesäuremethylester entsprechend einer Ausbeute von 67 % und den 3-Methoxy-2,4,5-trifluorbenzoesäuremethylester (C) entsprechend einer Ausbeute von lediglich 8,7 %, jeweils bezogen auf eingesetzte 3-Hydroxy-2,4,5-trifluorbenzoesäure.

Das Problem einer geringen Bildung von 3-Methoxy-2,4,5-benzoesäuremethylester kann durch Zugabe einer geringen Menge (2 g) des in Beispiel 2b) verwendeten Gemisches verschiedener wasserunlöslicher Trialkylamine gelöst werden.

Man setzt zu der aus dem vorstehend beschriebenen Vergleichsbeispiel erhaltenen Reaktionsmischung 2 g des in Beispiel 2 b) genannten Gemisches von Trialkylaminen zu und erwärmt auf 40°C. Über einen Tropftrichter gibt man über einen Zeitraum von 2 Stunden 18,7 g (0,168 mol) Dimethylsulfat zu und hält durch Zugabe von 6,4 g einer 10 gew.-%igen wäßrigen Natronlauge den pH-Wert auf 7.

Danach kann die 3-Hydroxy-2,4,5-trifluorbenzoesäure nicht mehr nachgewiesen werden. Es haben sich allerdings 15,6 g (70,9 mmol) 3-Methoxy-2,4,5-trifluorbenzoesäuremethylester, entsprechend einer Ausbeute von nicht weniger als 87 % der Theorie gebildet.

Die im Vergleich zu Beispiel 2 b) etwas niedrigere Ausbeute ist auf die Vorbehandlung des Reaktionsgemisches zurückzuführen.

### Beispiel 3

a) Herstellung von 2,3,4,5-Tetrafluorbenzoesäure durch Decarboxylierung von Tetrafluorphthalsäure
   Die Umsetzung verläuft nach folgender Gleichung: 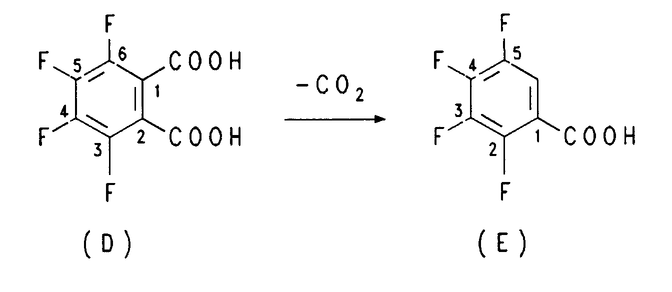
   In einem Glaskolben werden unter Rühren 2206 g einer wasserhaltigen Mischung (Wassergehalt 8,3 Gew.-%), die 311 g (1,31 mol) Tetrafluorphthalsäure (D) in einem Gemisch von 150 g Trialkylaminen mit 8 bis 10 Kohlenstoffatomen je Alkylrest (Hostarex A327), 100 g Diphenylmethan und 300 g Xylol enthält, vorgelegt.
   Man stellt den pH-Wert mit 96 %iger Schwefelsäure auf 6 bis 7 ein und erhitzt 9 Stunden unter kräftigem Rückfluß auf 110°C.
   Anschließend erhitzt man unter gutem Rühren auf 110°C und destilliert im Verlauf von 2 Stunden 97,8 g Wasser und 179,1 g Xylol ab. Das nach Abdestillieren verbleibende Reaktionsgemisch enthält 234,9 g (1,21 mol entsprechend 92,4 % der Theorie) 2,3,4,5-Tetrafluorbenzoesäure (E), bestimmt mittels kalibrierter HPLC-Analyse.
b) Herstellung von 2,3,4,5-Tetrafluorbenzoesäureethylester
   Die Umsetzung verläuft nach folgender Gleichung 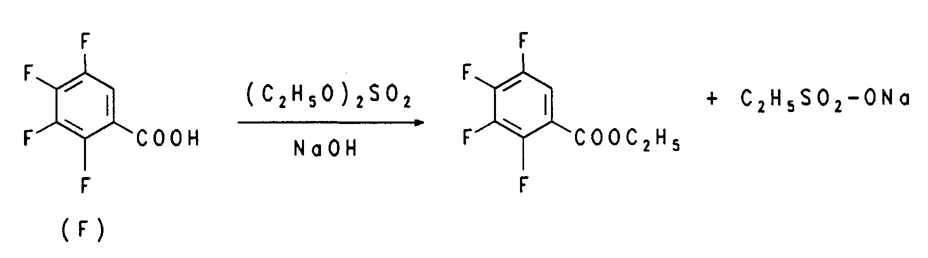
   Man versetzt das aus Beispiel 3a) erhaltene Reaktionsgemisch mit dem in Beispiel 3a) abgetrennten Destillat (97,8 g Wasser und 179,1 g Xylol). Anschließend tropft man unter Rühren bei einer Temperatur von 40°C über einen Zeitraum von 4 Stunden 856,9 g (5,55 mol) Diethylsulfat und hält durch Zugabe von wäßriger Natronlauge den pH-Wert in einem Bereich von 7 bis 8. Man rührt 75 Minuten nach, setzt 10 g Ammoniumchlorid zu und rührt 2 Stunden nach. Man filtriert bei pH 8,2 ab und trennt unter Zugabe von 600 g Xylol und 1000 g Diphenylmethan die wäßrige von der organischen Phase. Aus der organischen Phase (2330 g) werden durch Destillation bei einer Temperatur bis 193°C und bei einem reduzierten Druck bis 4 bis 5 mbar (2 bis 3 Torr) 1031,7 g Destillat erhalten, das, bestimmt mittels kalibrierter gaschromatographischer Analyse, 245 g (1,1 mol) 2,3,4,5-Tetrafluorbenzoesäureethylester, entsprechend einer Ausbeute von 84 % über 2 Stufen, bezogen auf eingesetzte Tetrafluorphthalsäure enthält.
   Arbeitet man anstatt mit dem Trialkylamin-Gemisch (Hostarex A 327) mit 200 g Chinolin, so verläuft der Ansatz völlig analog. Man erhält, bestimmt mittels kalibrierter HPLC-Analyse, 234,5 g (1,07 mol entsprechend 81,3 % der Theorie) 2,3,4,5-Tetrafluorbenzoesäureethylester.
   Unter Verwendung von 300 g Collidin beträgt die Ausbeute an 2,3,4,5-Tetrafluorbenzoesäureethylester 82,1 % der Theorie.

### Beispiel 4

### Herstellung von 3-Methoxy-2,4,5-trifluorbenzoesäuremethylester (Zusatz eines Phasentransferkatalysators zur Unterstützung der Durchmischung)

Die Umsetzung folgt der in Beispiel 2b genannten Gleichung durch Veresterung von 3-Hydroxy-2,4,5-trifluorbenzoesäure (B).

1 kg einer wäßrigen, analog zu Beispiel 2a hergestellten Lösung, die von den wasserunlöslichen Aminen abgetrennt wurde, wird unter Rühren mit 10 gew.-%iger wäßriger Natronlauge versetzt, bis ein pH-Wert von 7,5 eingestellt ist. Das Gemisch enthält 54,6 g (0,284 mol) 3-Hydroxy-2,4,5-trifluorbenzoesäure (B), bestimmt durch HPLC Analyse (Kalibrierung mit externem Standard). Man setzt unter Rühren mittels eines Magnetrührers 20 g Hostarex A 327 (siehe auch Beispiel 2b) und 20 g einer 50 %igen wäßrigen Lösung eines Dimethylbenzylcocosalkyl-(C₁₀-C₁₈)-ammoniumchlorids mit einem durchschnittlichen Molgewicht von 382,5 (Dodigen 226) zu. Anschließend erwärmt man auf 50 bis 52°C und läßt binnen 4,5 Stunden 329,2 g (2,61 mol) Dimethylsulfat zutropfen und hält durch Zugabe von 10 gew.-%iger wäßriger Natronlauge den pH-Wert der gerührten Mischung bei 7 bis 7,5 (Verbrauch 133 g).
Der pH-Wert der das Wasser, das Gemisch wasserunlöslicher Trialkylamine mit 6 bis 8 Kohlenstoffatomen je Alkylrest (Hostarex A 327) und die 3-Hydroxy-2,4,5-fluorbenzoesäure enthaltende Mischung wird mittels einer geeichten pH-Elektrode, die in die wäßrige Phase eintaucht, kontrolliert.

Die Umsetzung ist nach einer Nachrührdauer von 2 Stunden bei pH 7 bis 7,5 und 50 bis 52°C beendet (bestimmt durch HPLC Analyse). Man gibt 50 g Xylol zu und trennt die organische Phase ab. Dieser Schritt wird zweimal wiederholt.

Die vereinigten organischen Phasen enthalten (bestimmt durch kalibrierte HPLC-Analyse) 55,5 g (0,252 mol) 3-Methoxy-2,4,5-trifluorbenzoesäuremethylester, entsprechend einer Ausbeute von 88,7 % bezogen auf eingesetzte 3-Hydroxy-2,4,5-trifluorbenzoesäure (B).

## Patentansprüche

1. Verfahren zur Herstellung von Verbindungen der Formel (1)
R¹R²R³R⁴R⁵ArCOOR (1)
worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OR, NHR, NR₂, SR oder COOR, wobei R ein Alkylrest mit 1 bis 4 Kohlenstoffatomen ist, stehen, Ar für einen Arylrest mit 6 bis 12 Kohlenstoffatomen steht und der in Formel (1) ausgewiesene Rest R die vorstehende Bedeutung hat, dadurch gekennzeichnet, daß man eine Verbindung der Formel (2)
R¹R²R³R⁴R⁵ArCOOH (2)
worin R¹, R², R³, R⁴, R⁵ gleich oder verschieden sind und für Wasserstoff, ein Halogen, eine Alkyl- oder Alkoxygruppe mit 1 bis 6 Kohlenstoffatomen, für OH, NH₂, NHR, SH oder COOH stehen und Ar dieselbe Bedeutung wie in Formel (1) hat, mit einem Sulfat der Formel (RO)₂SO₂, worin R die vorstehende Bedeutung hat, in Anwesenheit eines wasserunlöslichen tertiären Amins und Wasser bei einer Temperatur von 10 bis 120°C in Anwesenheit oder Abwesenheit eines wasserunlöslichen Lösungsmittels und unter Zugabe einer Base bei einem pH-Wert von 5 bis 12 umsetzt.

2. Verfahren nach Anspruch 1, dadurch gekennzeichnet, daß man eine Verbindung der Formel (2) worin R¹ und R² gleich oder verschieden sind und für Wasserstoff, eine Alkylgruppe mit 1 bis 6 Kohlenstoffatomen, für OH, NH₂ oder COOH stehen, einsetzt.

3. Verfahren nach Anspruch 1 oder 2, dadurch gekennzeichnet, daß man eine Verbindung der Formel (2), worin R¹ und R² gleich oder verschieden sind und für Wasserstoff, für OH oder COOH stehen, einsetzt.

4. Verfahren nach einem oder mehreren der Ansprüche 1 bis 3, dadurch gekennzeichnet, daß man eine Verbindung der Formel (2), worin R¹ oder R² für OH oder COOH stehen, einsetzt.

5. Verfahren nach einem oder mehreren der Ansprüche 1 bis 4, dadurch gekennzeichnet, daß man eine Verbindung der Formel (2), worin R¹ oder R² für OH steht, einsetzt.

6. Verfahren nach einem oder mehreren der Ansprüche 1 bis 5, dadurch gekennzeichnet, daß man eine Verbindung der Formel (2), worin R³, R⁴ und R⁵ gleich oder verschieden sind und für Wasserstoff, ein Halogen, eine Alkyl- oder Alkoxygruppe mit 1 bis 6 Kohlenstoffatomen stehen, einsetzt.

7. Verfahren nach einem oder mehreren der Ansprüche 1 bis 6, dadurch gekennzeichnet, daß man eine Verbindung der Formel (2), worin R³, R⁴ und R⁵ gleich oder verschieden sind und für Wasserstoff, Fluor, Chlor oder eine Alkylgruppe mit 1 bis 6 Kohlenstoffatomen steht, einsetzt.

8. Verfahren nach einem oder mehreren der Ansprüche 1 bis 7, dadurch gekennzeichnet, daß man eine Verbindung der Formel (2), worin R³, R⁴ und R⁵ gleich oder verschieden sind und für Wasserstoff oder Fluor stehen, einsetzt.

9. Verfahren nach einem oder mehreren der Ansprüche 1 bis 8, dadurch gekennzeichnet, daß man eine Verbindung der Formel (2), worin Ar für einen Phenylrest, Biphenylrest oder Naphthylrest steht, einsetzt.

10. Verfahren nach einem oder mehreren der Ansprüche 1 bis 9, dadurch gekennzeichnet, daß man eine Verbindung der Formel (2), worin Ar für einen Phenylrest steht, einsetzt.

11. Verfahren nach einem oder mehreren der Ansprüche 1 bis 10, dadurch gekennzeichnet, daß man als Sulfat der Formel (RO)₂SO₂ Dimethylsulfat, Diethylsulfat oder Dibutylsulfat einsetzt.

12. Verfahren nach einem oder mehreren der Ansprüche 1 bis 11, dadurch gekennzeichnet, daß man als Sulfat der Formel (RO)₂SO₂ Dimethylsulfat oder Diethylsulfat einsetzt.

13. Verfahren nach einem oder mehreren der Ansprüche 1 bis 12, dadurch gekennzeichnet, daß man das Sulfat der Formel (RO)₂SO₂, bezogen auf jede in die Verbindung der Formel (2) einzuführende Gruppe R im Verhältnis (1 bis 10):1, insbesondere (1,1 bis 5):1, bevorzugt (1,2 bis 1,5):1 einsetzt.

14. Verfahren nach einem oder mehreren der Ansprüche 1 bis 13, dadurch gekennzeichnet, daß man als wasserunlösliches tertiäres Amin ein Trialkylamin mit 4 bis 20 Kohlenstoffatomen je Alkylrest, ein Gemisch dieser Trialkylamine, eine N-enthaltende heterocyclische Verbindung oder ein Gemisch der vorstehenden Amine einsetzt.

15. Verfahren nach einem oder mehreren der Ansprüche 1 bis 14, dadurch gekennzeichnet, daß man als wasserunlösliches tertiäres Amin ein Trialkylamin mit 6 bis 14 Kohlenstoffatomen je Alkylrest oder ein Gemisch dieser Trialkylamine einsetzt.

16. Verfahren nach einem oder mehreren der Ansprüche 1 bis 15, dadurch gekennzeichnet, daß man als wasserunlösliches tertiäres Amin ein Gemisch von Trialkylaminen mit 6 bis 12 Kohlenstoffatomen je Alkylrest, die gleiche oder verschiedene geradkettige oder verzweigte Alkylreste enthalten, einsetzt.

17. Verfahren nach einem oder mehreren der Ansprüche 1 bis 16, dadurch gekennzeichnet, daß man das wasserunlösliche tertiäre Amin und die Verbindung der Formel (2) im Molverhältnis (0,01 bis 10):1, insbesondere (0,05 bis 3):1, bevorzugt (0,05 bis 1):1 einsetzt.

18. Verfahren nach einem oder mehreren der Ansprüche 1 bis 17, dadurch gekennzeichnet, daß man die Umsetzung bei 20 bis 80°C durchführt.

19. Verfahren nach einem oder mehreren der Ansprüche 1 bis 18, dadurch gekennzeichnet, daß man die Umsetzung bei 30 bis 60°C durchführt.

20. Verfahren nach einem oder mehreren der Ansprüche 1 bis 19, dadurch gekennzeichnet, daß man als wasserunlösliches Lösungsmittel einen aliphatischen Kohlenwasserstoff, einen halogenierten aliphatischen Kohlenwasserstoff, einen aromatischen Kohlenwasserstoff, einen halogenierten aromatischen Kohlenwasserstoff, einen aromatischen Ether oder ein Gemisch dieser Lösungsmittel einsetzt.

21. Verfahren nach einem oder mehreren der Ansprüche 1 bis 20, dadurch gekennzeichnet, daß man als Base eine wäßrige Lösung eines Alkalihydroxids oder Erdalkalihydroxids einsetzt.

22. Verfahren nach einem oder mehreren der Ansprüche 1 bis 21, dadurch gekennzeichnet, daß man die Umsetzung bei einem pH-Wert von 6 bis 10 durchführt.

23. Verfahren nach einem oder mehreren der Ansprüche 1 bis 22, dadurch gekennzeichnet, daß man die Umsetzung bei einem pH-Wert von 7 bis 8,5 durchführt.

24. Verfahren nach einem oder mehreren der Ansprüche 1 bis 23, dadurch gekennzeichnet, daß man die Umsetzung in Anwesenheit eines Phasentransferkatalysators durchführt.

25. Verfahren nach einem oder mehreren der Ansprüche 1 bis 24, dadurch gekennzeichnet, daß man als Phasentransferkatalysator ein quartäres Ammoniumsalz der allgemeinen Formel 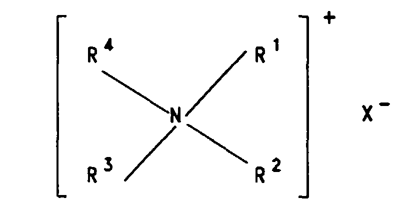 worin R¹, R², R³ und R⁴ gleich oder verschieden sind und für Kohlenwasserstoffreste mit insgesamt 10 bis 50 Kohlenstoffatomen stehen und X⁻ein Halogenidion, Hydrogensulfation oder Hydroxylion bedeutet, einsetzt.

26. Verfahren nach einem oder mehreren der Ansprüche 1 bis 25, dadurch gekennzeichnet, daß man als Phasentransferkatalysator ein Tetra(C₁-C₂₀)alkylammoniumsalz, Tri(C₁-C₂₀)alkylbenzylammoniumsalz, Di(C₁-C₂₀)alkyldibenzylammoniumsalz, deren Benzylrest, unsubstitutiert oder durch Cl, Br, (C₁-C₄)Alkyl, (C₁-C₄)Alkoxy substituiert ist, einsetzt.

27. Verfahren nach einem oder mehreren der Ansprüche 1 bis 26, dadurch gekennzeichnet, daß man als Phasentransferkatalysator ein einen oder mehrere Cocos(C₁₀-C₁₈)alkylreste enthaltendes quartäres Ammoniumhalogenid einsetzt.

28. Verfahren nach einem oder mehreren der Ansprüche 1 bis 27, dadurch gekennzeichnet, daß man als Phasentransferkatalysator ein Dicocos(C₁₀-C₁₈)alkyldimethylammoniumhalogenid oder Dimethylbenzylcocos(C₁₀-C₁₈)alkylammoniumhalogenid einsetzt.

29. Verfahren nach einem oder mehreren der Ansprüche 1 bis 28, dadurch gekennzeichnet, daß man den Phasentransferkatalysator in einer Menge von 0,05 bis 10 Gew.-%, bezogen auf die wäßrige Phase einsetzt.
